(19) Europäisches Patentamt
European Patent Office
Office européen des brevets

(11) Veröffentlichungsnummer : **0 043 420**
**B1**

# (12) EUROPÄISCHE PATENTSCHRIFT

(45) Veröffentlichungstag der Patentschrift : **09.03.83**

(51) Int. Cl.³ : **C 07 C 41/01**// C07C93/04

(21) Anmeldenummer : **81103411.5**

(22) Anmeldetag : **06.05.81**

## (54) Verfahren zur Herstellung von methylblockierten Ethoxylaten.

(30) Priorität : **03.07.80 DE 3025190**

(43) Veröffentlichungstag der Anmeldung : **13.01.82 Patentblatt 82/02**

(45) Bekanntmachung des Hinweises auf die Patenterteilung : **09.03.83 Patentblatt 83/10**

(84) Benannte Vertragsstaaten : **AT BE FR GB IT NL**

(56) Entgegenhaltungen : **FR A 2 387 202**

(73) Patentinhaber : **CHEMISCHE WERKE HÜLS AG**
**Postfach 1320**
**D-4370 Marl 1 (DE)**

(72) Erfinder : **Brockhaus, Rudolf, Dr.**
**Holsteiner Strasse 19**
**D-4370 Marl (DE)**
Erfinder : **Franke, Hans-Jürgen**
**Freiheitstrasse 13**
**D-4270 Dorsten (DE)**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**EP 0 043 420 B1**

Jouve, 18, rue St-Denis, 75001 Paris, France

Verfahren zur Herstellung von methylblockierten Ethoxylaten

Monoalkyl- bzw. Monoaryl-ethoxylate, die Monoalkyl- bzw. Monoarylether von Polyglykolen sind ebenso wie die Ethoxylate, die Polyglykole, leicht herzustellen. Sie werden technisch aus Alkoholen bzw. Phenolen und Ethylenoxid hergestellt. Durch Einsatz von Ethylenoxid und Propylenoxid baut man auch gemischte Etherketten auf. Die Eigenschaften dieser Verbindungen hängen ab von der Struktur des endständigen Alkohols, von der Zahl der ankondensierten Ethoxyl- bzw. Propoxylgruppen sowie bei Polyglykolen vom Molekulargewicht.

Die Polyglykole und deren Mono-ether eignen sich aufgrund ihrer anwendungstechnischen Eigenschaften als nichtionische Tenside, Lösemittel für Gaswaschung oder Hydraulikflüssigkeit usw., aber die OH-Gruppe bewirkt eine gewisse Instabilität, z. B. gegen Oxidationen. Diesen Nachteil haben die Dialkylether nicht. So leicht und variabel der Aufbau von Polyglykolen (1,2-Glykolen) und von deren Monoalkylethern ist, so schwierig ist die Herstellung von den Dialkylethern.

Dialkylethoxylate erhält man beispielsweise durch Umsetzung von Monoalkylethoxylaten mit Isobuten, wobei ein t-Butylalkyl-ethoxylat entsteht. Ebenso lassen sich Methylether nach klassischen Methoden wie durch Umsetzung des Na-alkoholates mit Halogenkohlenwasserstoffen gewinnen. Diese Herstellungsmethoden sind jedoch sehr aufwendig.

Aus der US-PS 3 428 692 ist es bekannt, durch Erhitzen von aus vicinalen Glykolen bestehenden Ethoxylaten oder deren Monoalkyl- bzw. Monophenylethern auf 200 bis 300 °C in Gegenwart von Nickel- oder Cobalt-Katalysatoren, vorzugsweise Raney-Nickel, durch Deformylierung Methylether, die methylblockierten Ethoxylate, herzustellen. Dabei entstehen jedoch Gemische von den gewünschten Methylethern mit nicht vollständig umgesetzten Ethoxylaten und noch nicht identifizierten Aldehydverbindungen nach folgendem Reaktionsschema :

$$R{-}O{-}CH_2CH_2OH \xrightarrow[\Delta H]{\text{Ni od. Co}} R{-}O{-}CH_2CHO + H_2$$

$$R{-}O{-}CH_2CHO \longrightarrow R{-}O{-}CH_3 + CO$$

Daher besteht ein großes Interesse, diese Monoalkyl- bzw. Monoaryl-methyl-ethoxylate und Dimethylethoxylate selektiv und in hohen Ausbeuten herzustellen.

Die sich hieraus ergebende Aufgabe wurde erfindungsgemäß entsprechend den Angaben der Ansprüche gelöst.

Überraschenderweise erhält man durch Erhitzen der Ethoxylate, der Alkyl- bzw. Aryl-ethoxylate oder der Mono- bzw. Di-alkylamin- bzw. arylamin-ethoxylate in Gegenwart von Palladium-, Platin- und/oder Rhodium-Katalysatoren auf 180 bis 320 °C die methylblockierten Ethoxylate wie die Monoalkyl- bzw. Monoarylmethyl-ethoxylate und Dimethylethoxylate selektiv in nahezu quantitativen Ausbeuten. Auch bei unvollständigem Umsatz entsteht praktisch kein Aldehyd. Aus Ethoxylaten erhält man die Dimethylethoxylate mit einem um 2 HCHO (bzw. 2 $H_2$ + 2 CO) erniedrigten Molekulargewicht, aus Mono-alkylethoxylaten bzw. den entsprechenden Aryl-, Mono- bzw. Dialkylamin- oder arylamin-derivaten die Monoalkyl- (bzw. aryl-, alkylamin- oder arylamin-) methylethoxylate mit einem um 1 HCHO (bzw. $H_2$ + CO) erniedrigten Molekulargewicht.

Als Ethoxylate eignen sich die Ethoxylate mit 3 oder mehr Ethoxylgruppen, das Triethylenglykol (Triglykol) sowie die höhermolekularen Ethoxylate.

Bei diesen Ethoxylaten handelt es sich im allgemeinen nicht um völlig einheitliche Produkte. Ein Ethoxylat mit 8 Ethoxylgruppen hat beispielsweise im Mittel 8 Ethoxylgruppen, daneben aber auch teilweise 6 und 7 sowie 9 und 10 Ethoxylgruppen.

Außer den reinen Ethoxylaten eignen sich auch die aus Ethylenoxid und Propylenoxid aufgebauten gemischten Ethoxylate-propoxylate. Das Diglykol wird nur teilweise zum Methylglykol umgesetzt, teilweise reagiert Diglykol unter Ringschluß und Wasserabspaltung zu Dioxan.

Als Monoalkyl- bzw. -aryl-ethoxylate mit zwei oder mehr Ethoxylgruppen eignen sich beispielsweise die Monomethyl-, -ethyl-, -propyl-, -butyl-, -hexyl-, -octyl-, -lauryl-, -phenyl- und -alkylphenyl-ethoxylate. Geeignete Mono- bzw. Di-alkylamin- bzw. -arylaminethoxylate mit zwei oder mehr Ethoxylgruppen sind beispielsweise Methylamin-, Dimethylamin-, Ethylamin-, Diethylamin-, Butylamin-, Laurylamin-, Anilin-, Methylanilinethoxylate.

Tert. Monoalkyl-ethoxylate und tert. Monoalkylaminethoxylate sind weniger geeignet, da sie zum Zerfall neigen.

Als Katalysatoren eignen sich Palladium-, Platin- oder Rhodiumkatalysatoren wie auch Mischungen dieser Katalysatoren. Wegen des Mangels an diesen Edelmetallen setzt man jedoch bevorzugt auf Träger angebrachte Palladium-, Platin- und/oder Rhodium-Katalysatoren ein. Sehr gute Ergebnisse erzielt man mit Palladium, Platin oder Rhodium auf $Al_2O_3$ und/oder $SiO_2$, Carborund oder Aktivkohle als Träger. Die Träger enthalten im allgemeinen 0,1 bis 1 % Palladium, Platin und/oder Rhodium. Bevorzugt setzt man Palladiumkatalysatoren ein.

Die Umsetzung an den Palladium-, Platin- und/oder Rhodium-Katalysatoren erfolgt bei Temperaturen von 180 bis 320 °C, vorzugsweise von 210 bis 310 °C, insbesondere von 240 bis 300 °C. Die Reaktion führt man im allgemeinen bei Normaldruck durch, man kann jedoch im Unter- und Überdruckbereich arbeiten. Höhere Temperaturen führen zum Cracken des Moleküls.

Dabei entstehen sehr uneinheitliche Reaktionsprodukte und der Katalysator verliert seine Aktivität.

Die Reaktionszeit hängt ab von der Reaktionstemperatur und dem Verhältnis Reaktant zu Katalysator. Man erreicht Raumzeitausbeuten von etwa 40 bis 200 g/l Katalysator · Stunde. In Gegenwart von Palladiumkatalysatoren erreicht man Raumzeitausbeuten von 80 bis 200 g/l Katalysator · Stunde.

Von besonderem wirtschaftlichen Interesse ist das erfindungsgemäße Verfahren für die Umsetzung von schwer- oder nicht flüchtigen Produktionsrückständen der Ethoxylatsynthese, beispielsweise des Monoethylethoxylatrückstandes (Monoethylpolyglykoletherrückstand). Dieses Produkt läßt sich erfindungsgemäß in destillierbare Ethyl-methyl-ethoxylate überführen.

Die erfindungsgemäß erhaltenen methylblockierten Ethoxylate verwendet man vorzugsweise als Hydraulikflüssigkeit, beispielsweise als Bremsflüssigkeit. Man verwendet sie weiterhin als nichtionische Tenside mit besonders guter Beständigkeit gegen Luftsauerstoff.

## Beispiel 1

Umsetzung von Ethyldiglykol (Ethyl-ethoxylat) in Ethylmethylglykol (Ethylmethylethoxylat)

Als Reaktor dient ein Rohr von 32 mm Durchmesser und 80 cm Höhe. Der Reaktor ist gefüllt mit 600 ml eines Trägerkatalysators von 0,5 % Pd auf $Al_2O_3$-Träger, Körnung 3 bis 5 mm. Über eine Pumpe fährt man stündlich 69 g auf Reaktionstemperatur vorgewärmtes Ethyldiglykol ein. Die Reaktionstemperatur hält man bei 225 bis 230 °C, den Betriebsdruck bei 5 bar. Bei einem Ethyldiglykol-Umsatz von $> 99$ % (OH-Zahl im Reaktionsaustrag : 1,8) erhält man 52,4 g/h Ethylmethylglykol, was einer Raumzeitausbeute von 87,3 g Ethylmethylglykol/Liter Katalysator · Stunde entspricht.

## Beispiel 2

Umsetzung von Butyldiglykol (Butyl-ethoxylat) in Butylmethylglykol (Butylmethyl-ethoxylat)

In einem 1 l Rundkolben-Reaktor legt man 200 ml eines Trägerkatalysators von 0,5 % Pt auf $Al_2O_3$-Träger, 3 bis 5 mm Körnung, vor. Dazu gibt man 400 g Butyldiglykol. Durch Einleiten eines inerten Gasstromes von ca. 2 l/h wälzt man den Ansatz ständig leicht um.

Die Reaktionstemperatur hält man bei 190 bis 215 °C, den Druck bei 1 bar. Es entweicht ständig ein Gasgemisch von Wasserstoff, Kohlenmonoxid und Inertgas. Bei einem Butyldiglykol-Umsatz von ca. 45 % (OH-Zahl im Reaktionsprodukt : 208) ist ein Aldehydanteil von 0,8 % vorhanden (CO-Zahl : 2,8). Nach 36 Stunden liegt der Butyldiglykol-Umsatz bei $> 99$ % (OH-Zahl im Reaktionsprodukt : 2,7). Bei einer Ausbeute von 321 g Butylmethylglykol liegt die Leistung des Katalysators bei 44,6 g Butylmethylglykol/l Katalysator · Stunde.

## Beispiel 3

Umsetzung von Phenyldiglykolether (Phenyl-ethoxylat) in Methylphenylglykol (Methylphenyl-ethoxylat)

Reaktor ist wie in Beispiel 1 beschrieben. Man setzt jedoch 600 ml eines Trägerkatalysators von 0,5 % Rh auf $SiO_2$-Träger, Körnung 3 bis 5 mm ein. Über eine Pumpe fährt man stündlich 30,1 g Phenyldiglykolether ein. Die Reaktionstemperatur hält man bei 280 °C, den Betriebsdruck bei 1,5 bar. Dabei liegt der Phenyldiglykol-Umsatz bei $> 99$ % (OH-Zahl im Reaktionsaustrag : 3,1). Der Reaktionsaustrag enthält 24,6 g/h Methylphenylglykol. Hieraus ergibt sich eine Raumzeitausbeute von 41,1 g Methylphenylglykol/l Katalysator · Stunde.

## Beispiel 4

Umsetzung von Laurylethoxylat (im Mittel 8 Eo) in Laurylmethylethoxylat (Laurylheptaglykolmethylether)

In einem 1 l Rundkolben-Reaktor legt man 250 ml des in Beispiel 1 beschriebenen Katalysators und 500 g Laurylethoxylat (8 Eo) vor. Durch Einleiten von $\sim 2$ l/h Inertgas wälzt man den Ansatz ständig leicht um. Bei einer Reaktionstemperatur von 240 bis 250 °C und Normaldruck liegt der Laurylethoxylat-Umsatz nach 11 h $> 99$ % (OH-Zahl im Reaktionsprodukt : 0,9). Die Ausbeute von 467 g Laurylmethylheptaglykol entspricht einer Raumzeitausbeute von 169,8 g Laurylmethylheptaglykol/l Katalysator · Stunde.

## Beispiel 5

Umsetzung des Siederückstandes der Ethoxylatsynthese, Butylethoxylat (Mittel 5 Eo, Butylpolyglykolether) in Butylmethyltetraglykol.

Reaktor und Katalysator sind wie im Beispiel 1 beschrieben. Über eine Pumpe fährt man stündlich 92 g Siederückstand ein. Die Reaktionstemperatur hält man bei ca. 300 °C, den Betriebsdruck bei 1,5 bar. Dabei erhält man einen Butylpolyglykolether-Umsatz von > 99 % (OH-Zahl im Reaktionsaustrag : 2). Im gasförmigen Reaktoraustrag befinden sich neben Wasserstoff und Kohlenmonoxid Spuren von Methan. Die Ausbeute von 80,4 g/h Butylmethyltetraglykol entspricht einer Raumzeitausbeute von 134 g Butylmethyltetraglykol/l Katalysator · Stunde.

Beispiel 6

Umsetzung des Siederückstandes der Ethoxylatsynthese, Ethyltetraglykolether (Ethylethoxylat, im Mittel 4 Eo) in Ethylmethyltriglykol.

Reaktor und Katalysator sind wie im Beispiel 1 beschrieben.

Über eine Pumpe fährt man stündlich 83 g Siederückstand ein. Die Reaktionstemperatur hält man bei ca. 300 °C, den Betriebsdruck bei 1,5 bar. Dabei erhält man einen Ethyltetraglykolether-Umsatz von > 99 % (OH-Zahl im Reaktionsaustrag : 2,3). Der Reaktionsaustrag enthält 70,8 g Ethylmethyltriglykol, was einer Raumzeitausbeute von 118 g Ethylmethyltriglykol/1 Katalysator · Stunde entspricht.

Beispiel 7

Umsetzung von N,N-Bis-tetraethoxylat-talgfettamin

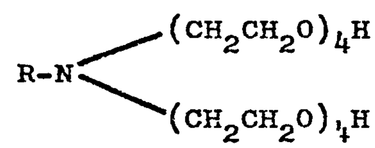

in N,N-Bis-methyltriglykol-talgfettamin.

In einem 1 l Rundkolben-Reaktor legt man 250 ml des in Beispiel 1 beschriebenen Katalysators und 500 g N,N-Bistetraethoxylat-talgfettamin vor. Bei einer Reaktionstemperatur von 245 °C und Normaldruck wird der Ansatz durch Einleiten von 2 l/h Inertgas ständig leicht umgewälzt. Nach 14 h liegt der N,N-Bis-tetraethoxylat-talgfettamin-Umsatz bei > 99 % (OH-Zahl im Reaktionsaustrag : < 1). Die Ausbeute beträgt 445 g N,N-Bis-methyltriglykol-talgfettamin, was einer Leistung des Katalysators von 127 g N,N-Bis-methyltriglykol-talgfettamin/l Katalysator · Stunde entspricht.

Beispiel 8

Umsetzung von Tetraglykol (Ethoxylat) in Dimethyldiglykol (Dimethylethoxylat)

Reaktor und Katalysator sind wie in Beispiel 1 beschrieben. Über eine Pumpe fährt man stündlich 120 g Tetraglykol ein. Die Reaktionstemperatur hält man bei 275 °C, den Betriebsdruck bei 1,5 bar.

Bei einem Tetraglykol-Umsatz von > 99 % (OH-Zahl im Reaktionsaustrag : 2,8) erhält man 81,9 g/h Dimethyl-diglykol, was einer Raumzeitausbeute von 136,5 g Dimethyldiglykol/l Katalysator · Stunde entspricht.

**Ansprüche**

1. Verfahren zur Herstellung von methylblockierten Ethoxylaten wie Monoalkyl-, Monoaryl-, Mono- bzw. Dialkylamin- bzw.-arylamin-methylethoxylaten und Dimethylethoxylaten durch Erhitzen von aus vicinalen Glykolen aufgebauten Ethoxylaten oder Monoalkyl-, Monoaryl-, Mono- bzw. Dialkylamin- bzw. -arylaminethoxylaten in Gegenwart von Katalysatoren, dadurch gekennzeichnet, daß man die Ethoxylate mit 3 oder mehr Ethoxylgruppen oder die Alkyl-, Aryl-, Mono- bzw. Dialkylamin- bzw. -arylaminethoxylate mit zwei oder mehr Ethoxylgruppen in Gegenwart von Palladium-, Platin- und/oder Rhodium-Katalysatoren auf 180 bis 320 °C erhitzt.

2. Verfahren nach Anspruch 1, dadurch gekennzeichnet, daß man auf Träger, insbesondere auf $Al_2O_3$- und/oder $SiO_2$-Träger, angebrachte Palladium-, Platin- oder Rhodium-Katalysatoren einsetzt.

3. Verfahren nach Anspruch 1, dadurch gekennzeichnet, daß man einen Palladium-Katalysator verwendet.

**Claims**

1. A process for the production of a methyl-terminated oxyethylate, such as an oxyethylate having one alkyl, aryl, mono- or di-alkylamino or mono- or di-arylamino terminal and one methyl terminal or an oxyethylate having two methyl terminals, by heating in the presence of a catalyst an oxyethylate derived from a vicinal glycol and optionally having one alkyl, aryl, mono- or di-alkylamino or mono- or di-arylamino terminal, characterised in that the oxyethylate has 3 or more oxyethyl groups and the oxyethylate having one alkyl, aryl, mono- or di-alkylamino or mono- or di-arylamino terminal has 2 or more oxyethyl groups and each is heated to 180 to 320 °C in the presence of a palladium, platinum and/or rhodium catalyst.

2. A process according to claim 1, characterised in that the palladium, platinum or rhodium catalyst is introduced in the form of a catalyst supported on a carrier, preferably an alumina and/or silica carrier.

3. A process according to claim 1, characterised in that a palladium catalyst is used.

**Revendications**

1. Procédé de préparation d'éthoxylats à blocage méthyle, tels que mono-alkyl-, mono-aryl-, mono- ou dialkyl-amine ou -arylamine-méthyl-éthoxylats et diméthyl-éthoxylats, par chauffage en présence de catalyseurs d'éthoxylats édifiés à partir de glycols vicinaux ou de mono-alkyl-, mono-aryl-, mono- ou di-alkylamine- ou -arylamine-éthoxylats, caractérisé par le fait qu'on chauffe les éthoxylats comportant trois groupes éthoxyle ou davantage, ou les alkyl-, aryl-, mono- ou di-alkyl-amine- ou -arylamine-éthoxylats qui comportent deux groupes éthoxyle ou davantage, à une température de 180 à 320 °C, en présence de catalyseurs au palladium, au platine et/ou au rhodium.

2. Procédé selon la revendication 1, caractérisé par le fait qu'on utilise des catalyseurs au palladium, au platine ou au rhodium apportés sur des supports, en particulier sur $Al_2O_3$ et/ou sur $SiO_2$.

3. Procédé selon la revendication 1, caractérisé par le fait qu'on utilise un catalyseur au palladium.